Europäisches Patentamt

European Patent Office

Office européen des brevets

(19)

(11) Publication number: **0 237 019**
**A2**

(12) **EUROPEAN PATENT APPLICATION**

(21) Application number: **87103406.2**

(22) Date of filing: **10.03.87**

(51) Int. Cl.³: **C 12 P 21/02**
**C 12 N 15/00, C 07 K 15/26**
**C 12 N 5/00, C 12 N 1/20**

(30) Priority: **14.03.86 JP 54650/86**

(43) Date of publication of application:
**16.09.87 Bulletin 87/38**

(84) Designated Contracting States:
**CH DE FR GB IT LI**

(71) Applicant: **TORAY INDUSTRIES, INC.**
**2, Nihonbashi-Muromachi 2-chome Chuo-ku**
**Tokyo 103(JP)**

(72) Inventor: **Tanaka, Toshiaki**
**31-22, Tsunishi 1-chome**
**Kamakura-shi Kanagawa(JP)**

(72) Inventor: **Kawano, Genji**
**3-13, Tsunishi 2-chome**
**Kamakura-shi Kanagawa(JP)**

(72) Inventor: **Sawada, Ritsuko**
**2-2-32, Katase**
**Fujisawa-shi Kanagawa(JP)**

(74) Representative: **Kador & Partner**
**Corneliusstrasse 15**
**D-8000 München 5(DE)**

(54) Interferon conjugate and production thereof using recombinant gene.

(57) An interferon conjugates comprising in a single molecule a region exhibiting biological activities of interferon-β and a region exhibiting biological activities of interferon-γ; deoxyribonucleotide sequences coding for the interferon conjugate; recombinant DNAs comprising the deoxyribonucleotide sequence and a deoxyribonycleotide sequence coding for a control region for expression of the interferon conjugate wherein the latter sequence is upstream from the former sequence; transformant organisms transformed with the recombinant DNA; and a process for production of the above-mentioned interferon conjugate comprising the steps of culturing the transformant organism to produce the interferon conjugate, and recovering the interferon conjugate.

Croydon Printing Company Ltd.

## INTERFERON CONJUGATE AND PRODUCTION THEREOF
## USING RECOMBINANT GENE

BACKGROUND OF THE INVENTION

1.  Field of the Invention

The present invention relates to interferon conjugates comprising interferon-β and interferon-γ, a process for production of these interferon conjugates, and a gene system including a nucleotide sequence coding for these interferon conjugate, a recombinant DNA comprising the nucleotide sequence, and microorganisms transformed with the recombinant DNA.

2.  Description of the Related Art

Interferons are proteins exhibiting various biological activities, such as antiviral activity, and therefore, are promising for clinical application.

Interferons are classified into three types, i.e., interferon-α, -β, and -γ, depending on their inducers, producer cells or antigenecities thereof, and are different in structure gene coding therefor, properties as protein, and in biological activities (Interferon no Kagaku (Science of Interferon), edited by Shigeyasu Kobayashi, Kodan Sha).

Interferon-β (IFN-β) is a glycoprotein usually produced by fibroblast induced with virus or doublestranded RNA, is stable to treatment at pH 2, and unstable to treatment at 56°C.  Gene coding for interferon-β has been already isolated (Taniguchi et al., Proc. Jpn. Acad., 55, Ser. B, 464-468, 1979).  The nucleotide sequence of the gene and a corresponding amino acid sequence have been determined, and moreover, a process for the production of interferon-β using cDNA thus obtained and E. coli as a host has been developed (Taniguchi et al., Proc. Natl. Acad. Sci. USA, 77, 5230-5233, 1980; Goeddel et al., Nucleic Acids Res., 8, 4057-4074, 1980; and Derynck et al., Nature, 287, 193-197, 1980).

Interferon-γ (IFN-γ) is a glycoprotein usually produced by T lymphocyte induced with a mitogen, and is unstable to treatment at pH 2. A gene coding for interferon-γ has been also isolated, the nucleotide sequence thereof has been determined, and a process for the production of interferon-γ using E. coli as a host has been established (Devos et al., Nucleic Acids Res. 10, 2487-2501, 1982; and Gray et al., Nature, 295, 503-508, 1982). Moreover, an amino acid sequence of native interferon-γ has been reported (Rinderknecht et al., J. Biol. Chem., 259, 6790-6797, 1984).

Among the interferons-α, -β, and -γ, interferons-α and -β have been known as interferon I, and it has been suggested that these interferons have a high structural analogy showing a 29% amino acid sequence homology (Taniguchi et al., Gene, 10, 11-15, 1980), and it is believed that they recognize same receptor. Therefore, the coexistence of interferons-α and -β provides their activities only in an additive manner. On the contrary, interferon-γ, known as interferon II, has a low amino acid homology with interferon I. It is believed that interferons I and II recognize different receptors (Branca et al., Nature, 294, 768-770, 1981). Therefore, interferons I and II exhibit different spectra of the anti-cell proliferation effect (Interferon no Kagaku (Science of Interferon), edited by Shigeyasu Kobayashi, Kodan Sha, Japan, 22-68, 1985), and exhibit their activities in a synergistic manner (Czarniecki et al., J. Virol., 49, 490-496, 1984; Fleishmann Jr. et al., J. IFN. Res., 4, 265-274, 1984; and Japanese Unexamined Patent Publication 59-98019).

Although a mixture of interferons-β and -γ will show the above-mentioned synergism in vitro, it is doubtful whether such synergism is established in vivo because there is some possibility that both interferon-β and -γ would not be simultaneously present at a target site due to their different _in vivo_ pharmacodynamics.

To resolve the above-mentioned disadvantage provided by the problems of _in vivo_ pharmacodynamics, it would be effective to link interferons-β and -γ to make a single polypeptide which could exhibit _in vivo_ the same synergism as shown _in vitro_ by a mixture of interferons-β and -γ. This means that, since a linked polypeptide molecule exhibits the same synergistically enhanced actions as shown by the parent two interferon molecules, i.e., -β and -γ, the activity per a molecule is enhanced in comparison to the activity per molecule of the parent interferons, making it possible to obtain a new type of interferon with a higher activity.

Moreover, by causing a single polypeptide to exhibit the different kinds of actions originally exhibited separately by interferons-β and -γ, it would be possible to obtain a new type of interferon having a broader spectrum of actions.

Some examples are already known wherein two polypeptides having different actions are linked to make a single polypeptide having two actions (Yourno et al., Nature, 228, 820-824, 1970; Neuberger et al., Nature, 312, 604-608, 1984; and Bulow et al., Biotechnology, 3, 821-823, 1985). Moreover, insulin molecules were linked to make a stable polypeptide (Shen and Shi-Hsiang, Proc. Natl. Acad. Sci. USA, 81, 4627-4631, 1984). As another example, there was disclosed a linkage of interferon-γ and interleukin-2 to make a single polypeptide which exhibits both actions of interferon-γ and interleukin-2 (Japanese Unexamined Patent Publication No. 60-241890).

However, so far there has been no attempt to link interferon-β with interferon-γ to prepare a single polypeptide having at the same time both a broader spectrum of actions and higher activities.

SUMMARY OF THE INVENTION

Therefore, the present invention relates to interferon conjugates comprising interferon-β and

interferon-γ, which have a broader spectrum of biological actions, such as antiviral action, anti-cell proliferation action, and the like, that were originally carried separately by parent polypeptides, i.e., interferon-β and interferon-γ, and which exhibits <u>in</u> <u>vivo</u> synergistically enhanced activities as shown <u>in</u> <u>vivo</u> by a mixture of interferons-β and -γ.

More specifically, the present invention provides an interferon conjugate comprising in a single molecule a region exhibiting biological activities of interferon-β and a region exhibiting biological activities of interferon-γ.

The present invention also provides a deoxyribonucleotide sequence coding for this interferon conjugate.

Moreover, the present invention provides a recombinant DNA comprising the above deoxyribonucleotide sequence and a deoxyribonucleotide sequence coding for a control region for expression of the interferon conjugate, wherein the latter sequence is present upstream from the former sequence.

Moreover, the present invention provides a transformant organism transformed with the recombinant DNA.

Moreover, the present invention provides a process for production of the above-mentioned interferon conjugate comprising the steps of culturing the transformant organism to produce the interferon conjugate, and recovering the interferon conjugate.

BRIEF DESCRIPTION OF THE DRAWINGS

Figure 1 represents an embodiment of an amino acid sequence of human mature interferon-β;

Fig. 2 represents an embodiment of an amino acid sequence of human mature interferon-γ;

Fig. 3 represents a structure of plasmid pKM6 for expression of human interferon-β;

Fig. 4 represents a structure of plasmid p6huγ-N1 for expression of human interferon-γ;

Fig. 5 represents a structure of plasmid pKM6-Cxho into which an XhoI site has been introduced at the 3'-terminus of the human interferon -β structural gen;

Fig. 6 represents a structure of plasmid p6huγNl-CKpn into which a KpnI site has been introduced at the 3'-terminus of the human interferon-γ structural gene;

Fig. 7 represents a structure of plasmid p6huγNlΔBS-NHin into which a HinPI site has been introduced to take out a human interferon-γ structural gene;

Fig. 8 schematically represents a process for construction of a plasmid for expression of an interferon-γ·β conjugate;

Fig. 9 schematically represents a process for construction of a plasmid for expression of an interferon-β·γ conjugate;

Fig. 10 schematically represents a process for construction of a plasmid for expression of an interferon-γcβ conjugate;

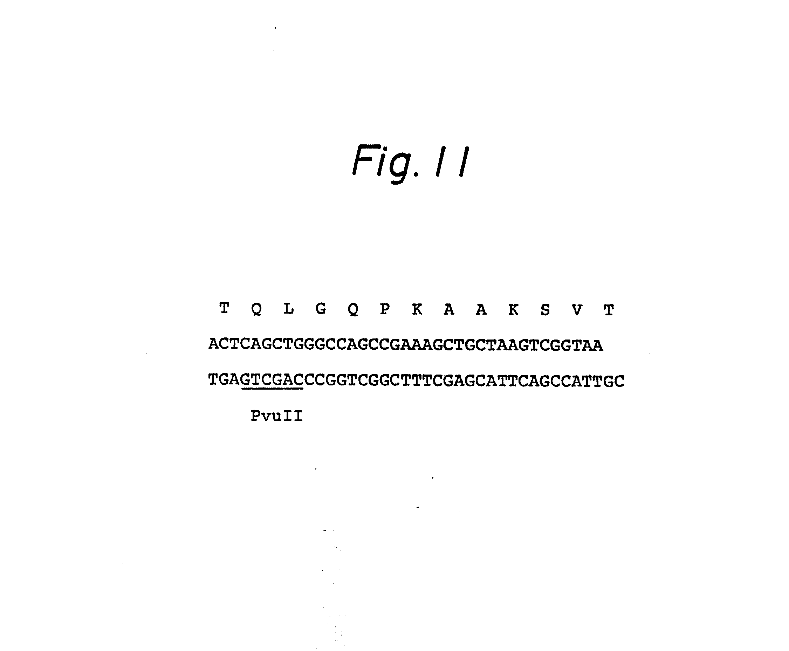

Fig. 11 represents a nucleotide sequence coding for an amino acid sequence of a spacer peptide;

Fig. 12 schematically represents a process for construction of a plasmid for expression of an interferon-βcγ conjugate;

Fig. 13 schematically represents a process for construction of a plasmid for expression of an interferon-γ·β conjugate in animal cells; and

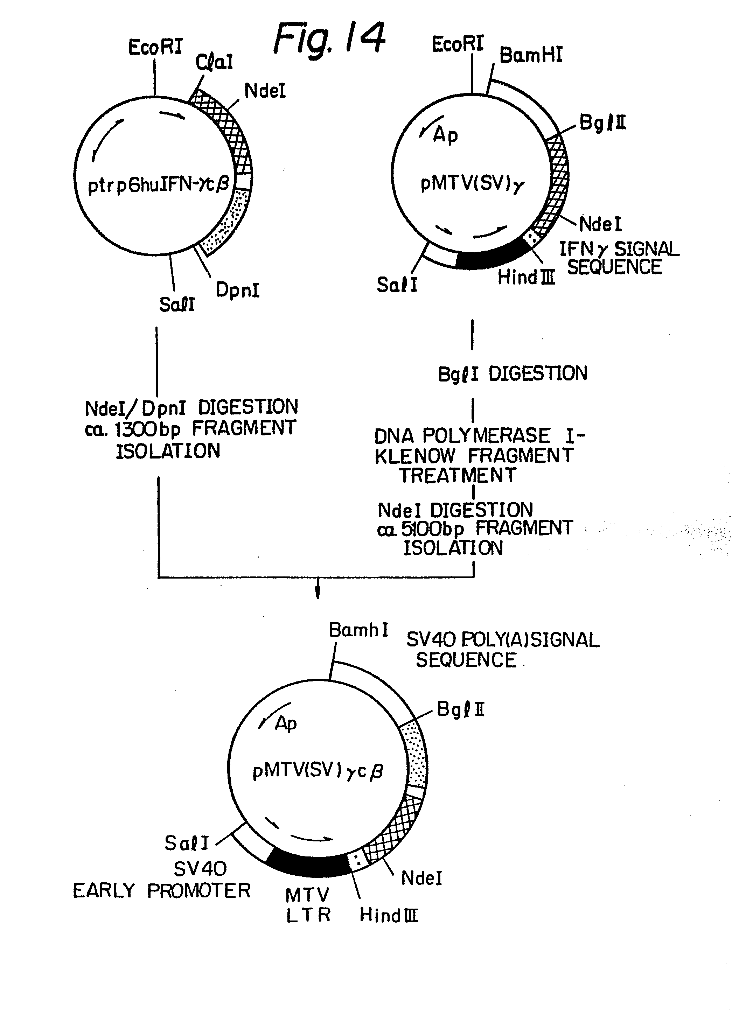

Fig. 14 schematically represents a process for construction of a plasmid for expression of an interferon-γcβ conjugate in animal cells.

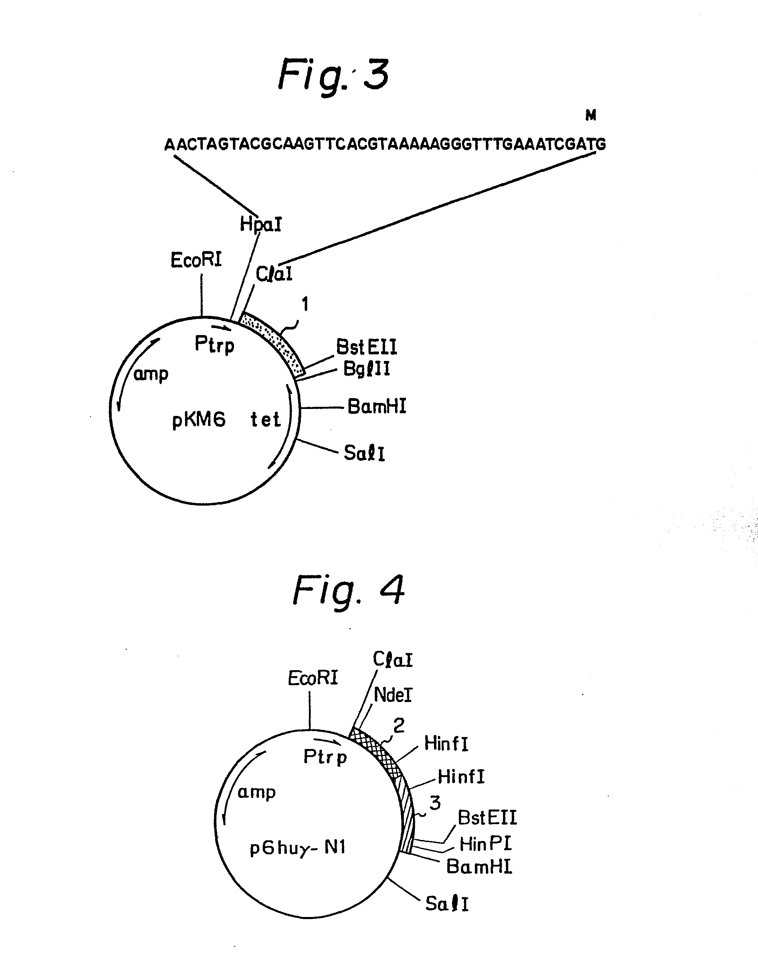

In Figures 3 to 10 and 12, the numbers 1, 2, and 3 represent a human interferon-β structural gene, a human interferon-γ structural gene, and a non-coding region of human interferon-γ cDNA, respectively.

DESCRIPTION OF THE PREFERRED EMBODIMENT

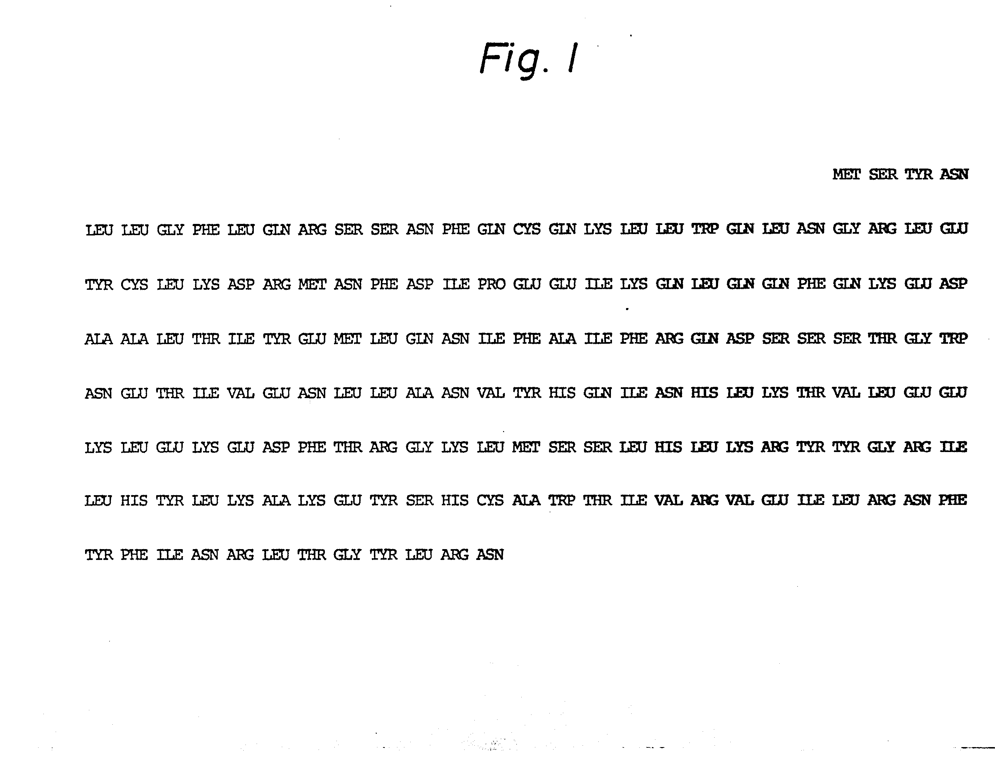

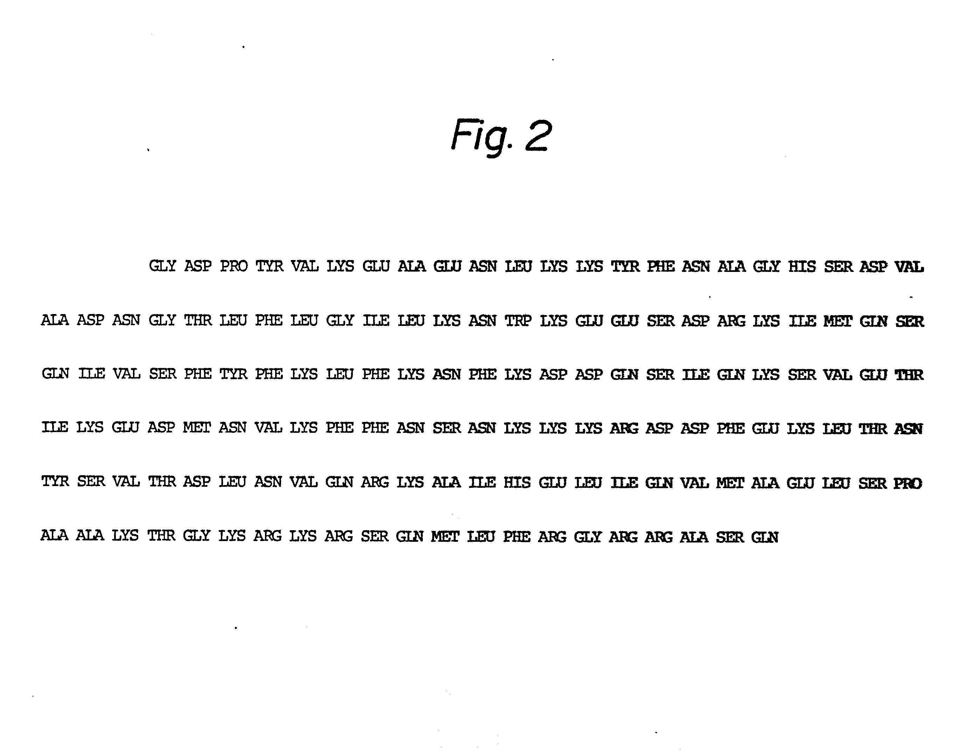

The interferon conjugate of the present invention comprises a region exhibiting biological activities of interferon-β and a region exhibiting biological

activities of interferon-γ linked directly or via a spacer. According to the present invention, the terms "interferon-β" and "interferon-γ" include any proteins having activities specific to interferon-β and interferon-γ, respectively. For example, for interferon-γ, a modified interferon-γ wherein its polypeptide has been extended by three amino acid residues at N-terminal (Gray et al., Nature, 295, 503-508, 1982), and a modified interferon-γ wherein a part of the C-terminal of the polypeptide thereof has been deleted (Rose et al., Biochem. J., 215, 273, 1983), are known. These modified interferon-γ fall under the scope of the present invention provided that they maintain interferon activities. Moreover, modified interferon-γ wherein some amino acid residues have been replaced by other amino acid residues are disclosed (Japanese Unexamined Patent Publications No. 59-93093 and No. 59-167596). These modified interferon-γ also fall under the scope of the present interferon-γ provided that they maintain interferon activities. Interferon-β also can be modified by the addition, deletion or replacement of one or more amino acid residues as described for interferon-γ. Preferably, interferon-β comprises a polypeptide having the amino acid sequence shown in Fig. 1, and interferon-γ comprises a polypeptide having the amino acid sequence shown in Fig. 2.

Juxtaposition order of interferons-β and -γ in the present interferon conjugate is not limited. That is, the interferon conjugate can contain as its N-terminal half a polypeptide of interferon-β and as its C-terminal half a polypeptide of interferon-γ, and vice versa.

In junction region in an interferon conjugate, a polypeptide of interferon-β and that of interferon-γ can be linked directly, or indirectly via a spacer, such as a peptide. The spacer peptide is preferably a peptide comprising many hydrophilic amino acids, as shown in an example for an enzyme wherein subunits of β-galactosidase are linked via a spacer peptide (Kushinke et al, EMBO J., 4, 1067-1073, 1985). Moreover, a polypeptide which links domains of a naturally occurring protein can be used as a spacer peptide of the present invention. A spacer peptide usually consists of up to 50 amino acids. A preferred spacer peptide is, for example, a peptide known as a switch-peptide of immunoglobulin molecule, and also a peptide having the amino acid sequence: Thr-Gln-Leu-Gly-Gln-Pro-Lys-Ala-Ala-Lys-Ser-Val-Thr.

In the present invention, a structure comprising a polypeptide of interferon-β and a polypeptide of interferon-γ is designated as "interferon conjugate". More specifically, a structure comprising as its N-terminal half a polypeptide of interferon-β and as its C-terminal half a polypeptide of interferon-γ is designated as "interferon-β·γ conjugate" (IFN-β·γ); and a structure comprising as its N-terminal half a polypeptide of interferon-γ, and at its C-terminal half, a polypeptide of interferon-β, is designated as "interferon-γ·β conjugate" (IFN-γ·β). On the other hand, a structure comprising as its N-terminal half a polypeptide of interferon-β and as its C-terminal half a polypeptide of interferon-γ wherein both polypeptides are linked via a spacer is designated as an "interferon-βcγ conjugate" (IFN-βcγ); and a structure comprising as its N-terminal half a polypeptide of interferon-γ and as its C-terminal half a polypeptide of interferon-β wherein both polypeptides are linked via a spacer is designated as an "interferon-γcβ conjugate" (IFN-γcβ).

As processes of the production of an interferon conjugate, there are a chemical synthesis wherein amino acids are coupled in a predetermined order, and genetic engineering process wherein a DNA coding for a target polypeptide is designed and constructed and the target polypeptide is expressed in appropriate transformed cells. In the present invention, preferably the genetic engineering is used because it enables an easy production

- 8 -

0237019

of a target polypeptide, though the means of obtaining a target polypeptide is not limited to genetic engineering.

In the use of genetic engineering for the production of an interferon conjugate, a DNA fragment comprising a nucleotide sequence coding for a polypeptide of interferon-β and a nucleotide sequence coding for a polypeptide of interferon-β, wherein these nucleotide sequences are directly linked or indirectly linked via a nucleotide sequence coding for a spacer peptide, are prepared, and this DNA fragment is linked to appropriate control regions for expression of the target polypeptide, and the coding sequence is expressed in appropriate host cells.

Any nucleotide sequence coding for an interferon conjugate can be used. That is, if more than one codon is present for an amino acid, any codon which codes for the amino acid can be used. Preferably, the same nucleotide sequences as those of the cDNAs of interferons-β and -γ respectively are used (Taniguchi et al., Gene, 10, 11-15, 1980; and Devos et al., Nucleic Acids Res., 10, 2487-2501, 1982).

To obtain a DNA fragment having a nucleotide sequence coding for an interferon conjugate, chemical synthesis of the DNA, isolation and linking of genes coding for interferons-β and -γ, and a combination of such means can be used. The chemical synthesis of a DNA fragment having a desired nucleotide sequence can be achieved according to a conventional procedure (Edge et al., Nature, 292, 756-762, 1981; and Tanaka et al., Nucleic Acids Res., 11, 1707-1723, 1983). DNA fragment coding for interferon-β or -γ can be derived from chromosomal DNA, or preferably, prepared as cDNA. cDNA can be isolated according to a known procedure (Taniguchi et al., Proc. Jpn. Acad., 55, Ser. B, 464, 1979; Goeddel et al., Nucleic Acids Res., 8, 4057-4074, 1980; Derynck et al., Nature, 287, 193-197, 1980; Devos et al., Nucleic Acids Res. 10, 2487-2501, 1982; and Gray et al., Nature, 295, 503-508, 1982). Alternatively, the desired DNA fragment can be obtained by screening a cDNA library prepared according to a known procedure (Okayama et al., Molecular and Cellular Biology, 3, 280, 1983) and using a probe designed from a known nucleotide sequence described in the above-mentioned literature.

To obtain a DNA fragment coding for an interferon conjugate from cDNAs of interferons-β and -γ, the cDNAs are separately digested with an appropriate restriction enzyme, and the resulting cDNA fragments are ligated immediately or after blunt-ending with a mung bean nuclease, DNA polymerase I Klenow fragment or T4 DNA polymerase. The cDNA fragments are preferably linked via a synthetic oligonucleotide corresponding to nucleotides deleted by the restriction enzyme cleavage, to obtain a DNA fragment coding for an interferon conjugate comprising entire polypeptides of interferons-β and -γ. In such a case, according to one embodiment of the present invention, the synthetic oligonucleotide consists of a nucleotide sequence coding for a spacer peptide flanked by nucleotide sequences corresponding to nucleotides deleted by the restriction enzyme cleavage, so as to obtain a DNA fragment coding for an entire interferon conjugate comprising polypeptides of interferons-β and -γ linked via the spacer peptide. Alternatively, a restriction enzyme site is introduced in cDNAs of interferon-β and interferon-γ at their 5'-end or 3'-end of the structural gene using synthetic oligonucleotides, and the cDNAs are cleaved with an appropriate restriction enzyme, and if necessary, after the cDNAs are blunt-ended, the cDNAs are ligated. In any case, one cDNA fragment should be linked in reading frame with another cDNA fragment.

To produce an interferon conjugate using the above-mentioned DNA construct, animal or plant cells, yeast or E. coli are used as a host. To express an interferon conjugate, the DNA construct should have a promoter for starting the transcription, and an SD sequence and ATG codon for starting the translation upstream therefrom. As a promoter, any nucleotide sequence having a promoter activity can be used; for example, lac promoter, trp promoter, recA promoter, and the like are known. Preferably a strong promoter such as trp promoter is used. The SD sequence is a ribosome RNA binding site and, therefore, essential for translation. In the present invention, any SD sequence can be used. A control region comprising the above-mentioned promoter and SD sequence thus constructed is added to a DNA fragment coding for an interferon conjugate via an ATG codon to express the interferon conjugate. The addition of the ATG codon can be carried out using synthetic oligonucleotides (Goeddel et al., Nature, 281, 544-548, 1979). Alternatively, for interferon-β, the ATG codon can be exposed according to a known procedure (Taniguchi et al., Proc. Natl. Acad. Sci. USA, 77, 5230-5233, 1980).

To introduce the thus-obtained DNA into host cells, a vector DNA is used. Vector DNAs used for an E. coli host include a plasmid DNA such as pBR322 and pSC101, and a phage DNA such as λ phage. Such a DNA vector is linked with the DNA construct for expression of an interferon conjugate to construct an expression DNA vector, which is then used to transform host cells according to a known procedure (Maniatis et al., "Molecular Cloning", Cold Spring Harbor Laboratory, p250-255, 1982).

A transformant such as transformed E. coli is then cultured in an appropriate medium to produce an interferon conjugate. Culturing is preferably carried out in a liquid medium. When trp promoter is used, indoleacrylic acid is added to a culture medium during culturing to induce the production of an interferon conjugate. If a different promoter is used, an inducer selected in accordance with the particular promoter is preferably used, to enhance the production of an interferon conjugate.

The interferon conjugate producer such as the thus-cultured E. coli cells are then disrupted according to a conventional cell disruption procedure ("Tanpakushitsu·Koso no Kisojikken" (Fundamental Experiment of Protein and Enzyme), edited by Takekazu Horio and Jinpei Yamashita, Nankodo, 1981, 3-7), for example, by treatment with a enzyme, sonication, grinding, or treatment under pressure, to obtain a crude extract containing an interferon conjugate.

The above-mentioned cell disruption procedure can be combined with treatment with guanidine hydrochloride or urea to improve the extraction efficiency (Davis et al., Gene, 21, 273-284, 1983).

The thus-obtained crude extract can be further purified according to a known procedure ("Tanpakushitsu·Koso no Kisojikken" (Fundamental Experiment of Protein and Enzyme), edited by Takekazu Horio and Jinpei Yamashita, Nankodo, 1981, 18-382), for example, by salting out, ultrafiltration, ion exchange, gel filtration, affinity chromatography, electrophoresis, or a combination thereof, to prepare a highly purified interferon conjugate preparation.

To express an interferon conjugate in animal cells, it is necessary that a nucleotide sequence coding for the interferon conjugate be present under the control of a promoter effective in the animal cells. Promoters effective in animal cells include, for example, SV40 early promoter, SV40 late promoter, a promoter of a HB virus gene, MMTV promoter, a promoter of a thymidine kinase gene, a promoter of a heat shock protein, and a promoter of an interferon gene. A nucleotide sequence coding for an interferon conjugate is linked downstream of one of the above-mentioned promoters in the same manner as described for the expression in E. coli. A single promoter or a combination of more than one promoter can be used. Note, an enhancer sequence of Harbey mouse sarcoma virus 5'LTR or an enhancer sequence of SV40 can be linked upstream of the above-mentioned promoter for a eukaryotic cell. Such enhancers are believed to increase the transcription efficiency. Preferably, a nucleotide sequence coding for signal peptide for extracellular secretion is added just upstream of the nucleotide sequence coding for an interferon conjugate, extracellularly to secrete the interferon conjugate, which can be then recovered in a supernatant of culture broth.

To prepare a large amount of the above-mentioned DNA construct so that the DNA construct can be easily introduced into animal cells, the DNA construct preferably includes an E. coli replication origin and a drug resistance gene. The replication origin is preferably, but not limited to, that derived from a colicin El plasmid, for example, pBR22, or a related plasmid. The drug resistance gene is, for example, an ampicillin resistant gene, tetracycline resistant gene, or kanamycin resistant gene. Moreover, the DNA construct can contain a replication origin responsible for autonomous replication in host cells, for example, a replication origin of SV40 virus or a replication origin of polyoma virus. These various DNA fragments are linked to construct an interferon conjugate expression vector.

The construction of a vector DNA can be carried out according to a conventional procedure (T. Maniatis et al., "Molecular Cloning", p86-96, 1982).

Animal cells into which a vector DNA will be introduced may be cells derived from humans, monkeys, Chinese hamsters, mice, etc., although since a human interferon conjugate is the target compound, human cells are preferably used. Human cells wherein growth is not inhibited by a peptide having glycoside chains produced are used. The human cells are preferably cells derived from human lung cancer, especially PC8 and PC12 cell lines (M. Kinjo et al., Br. J. Cancer, 39, 15, 1979).

Vector DNA can be introduced into host cells according to a known calcium phosphate method (F.L. Graham et al., Virology, 54, 536, 1973).

Cells into which a plasmid for expression of an interferon conjugate has been introduced can be efficiently obtained by cotransformation of host cells with the plasmid and G418 resistance gene expression vector pSV2neo (P.J. Southern et al., J. Mol. Appl. Genet., 1, 327, 1982) or pNE05' (M. Lusky et al., Cell, 36, 391, 1984), because the cotransformed cells can survive in a medium containing G418, in which non-cotransformed cells cannot survive.

The thus-obtained transformant is cultured in, for example, a medium containing fetal calf serum, extracellularly to produce an interferon conjugate, which is then recovered in a pure form from a supernatant of the cultured broth according to the same procedure as described for recovery from the E. coli extract. The thus-obtained interferon conjugate is a polypeptide having glycoside chains.

Since the interferon conjugate prepared as described above binds to an anti-human interferon-β antibody and to an anti-human interferon-γ antibody, it has the antigenecities of both interferons-β and -γ. Moreover, a neutralization test thereof with anti-human interferon-β antibody and anti-human interferon-γ antibody showed that a single polypeptide exhibits both interferon-β activity and interferon-γ activity.

As described above, the present invention provides a new type of interferon conjugate comprising a region exhibiting biological activities of interferon-β and a region exhibiting biological activities of interferon-γ, which is produced by recombinant cells containing DNA coding for interferon-β and interferon-γ.

The interferon conjugate of the present invention is a single polypeptide carrying biological actions which were originally carried separately on interferon-β and interferon-γ. Therefore, the present interferon conjugate has a broader action spectra, such as an antiviral spectrum and an anti-cell proliferation spectrum broader than those of conventional interferon. These properties make the present interferon conjugate valuable as an antiviral pharmaceutical or an antitumor pharmaceutical.

Moreover, since the present interferon conjugate exhibits the synergism conventionally provided by a mixture of interferon-β and interferon-γ, it provides remarkably enhanced activity per molecule in comparison to conventional interferons. Although such synergism can be obtained in vitro by only mixing interferon-β and -γ, since interferons-β and -γ, would show different in vivo pharmacodynamics, the coexistence of both interferons at a target site is not assured in vivo. This means that the mixture of both interferons-β and -γ would not exhibit the synergism. On the contrary, in the present interferon conjugate, since a single molecule provides the synergism, the problem of different pharmacodynamics does not exist. This also makes the present interferon conjugate valuable as an antiviral pharmaceutical and an antitumor pharmaceutical.

Finally, at present, to obtain interferon-β activity and interferon-γ activity, separate processes for the production of interferons-β and -γ must be carried out. On the contrary, according to the present invention, one production process provides an interferon conjugate carrying both interferon-β activity and interferon-γ activity. This gives an economical advantage to the present invention. The present interferon conjugate can be used as such, or can be easily cleaved into interferon-β and interferon-γ, which then can be used separately or as a mixture. In all cases the above-mentioned advantage still remains.

<u>Examples</u>

The present invention will now be further illustrated by, but is by no means limited to, the following examples.

Common procedures for gene manipuration used in the following Reference example and Examples are those described is Maniatis et al., "Molecular Cloning", Cold Spring Harbor Laboratory, 1982.

Prior to illustrating Examples of the present invention, the construction processes of a plasmid for the expression of human interferon-β and a plasmid for the expression of human interferon-γ are described as a Reference example.  These plasmids are used as starting plasmids for the construction of the present plasmids.

<u>Reference example</u>

(1) <u>Construction of plasmid pKM6 for expression of human interferon-β</u>.

A plasmid pTuIFNβ-5 for the expression of human interferon-β constructed according to a reported procedure (Taniguchi, Seikagaku, <u>54</u>, 363-377, 1982) was digested with HindIII, and the digestion product was treated with a T4 DNA polymerase Klenow fragment to make blunt ends, linked to a BglII linker, digested with BglII, and self-circularized using a T4 DNA ligase to obtain a plasmid pYO-10.  The plasmid pYO-10 was digested with SalI and ClaI, and the digestion product was subjected to agarose gel electrophoresis to isolate a DNA fragment of about 830 bp.  This DNA fragment was inserted into a ClaI-SalI region of the plasmid p6huγ-A2 described in Japanese Unexamined Patent Publication No. 61-19488 to construct a plasmid pKM6 shown in Fig. 3.

(2) <u>Construction of plasmid p6huγ-N1 for expression of human interferon γ</u>.

Lympocyte derived from human tensils was treated with phytohemagglutinin (PHA) and 12-o-tetradecanoyl-phorbor-13-acetate (TPA) to induce the production of human interferon-γ (Vilvek et al.,

Infection and Immunity, 34, 131, 1983), and then mRNA was prepared from the treated cells. The preparations of mRNA and cDNA, and the cloning of cDNA in a plasmid were carried out according to known procedures (Okayama et al., Molecular and Cellular Biology, 3, 280, 1983). The DNA library thus obtained was subjected to colony hybridization using a synthetic oligonucleotide probe having the sequence: 5'-AGGACAACCATTACT-3' corresponding to a region near the 3'-terminal of a known human interferon-γ structural gene (Goeddel et al., Nature, 295, 503-509, 1982) to obtain a plasmid pIFN-γ15 containing a cDNA coding for human interferon-γ. Next, after the plasmid pIFN-γ15 was digested with NdeI, and BamHI, a DNA fragment of about 0.9 kb was isolated by agarose gel electrophoresis.

On the other hand, two oligonucleotides 5'-CGATGCAGGACCCA-3' and 5'-TATGGGTCCTGCAT-3' were synthesized, and after pholpholyration at the 5'-end with a T4 polynucleotide kinase, the oligonucleotides were mixed to make a concentration of about 8 pmol/μl for each oligonucleotide. The mixture was then heated at 65°C for 3 munutes, cooled rapidly, heated again at 65°C for 3 minutes, and then allowed to stand at a room temperature to be cooled gradually and to anneal the oligonucleotides.

7 pmoles of this oligonucleotide, 0.3 pmole of NdeI-BamHI fragment from plasmid pIFNγ-15, and 0.1 pmole of a DNA fragment of about 4200 bp prepared by digestion of the plasmid pKM6 described in paragraph (1) with ClaI and BamHI followed by isolation by agarose gel electrophoresis were mixed, and after ligation the ligation mixture was used to transform E. coli MC1061 (Casadaban et al., J. Mol. Biol., 138, 179-207, 1980). The transformants selected for ampicillin resistance were subjected to colony hybridization using an oligonucleotide probe 5'-TATGGGTCCTGCAT-3', to obtain a plasmid p6hur-N1 for expression of human interferon-γ (Fig. 4).

Next, to link the interferon-β structural gene and interferon-γ structural gene, restriction enzyme sites are introduced to the 5'-end or 3'-end of each structural gene.

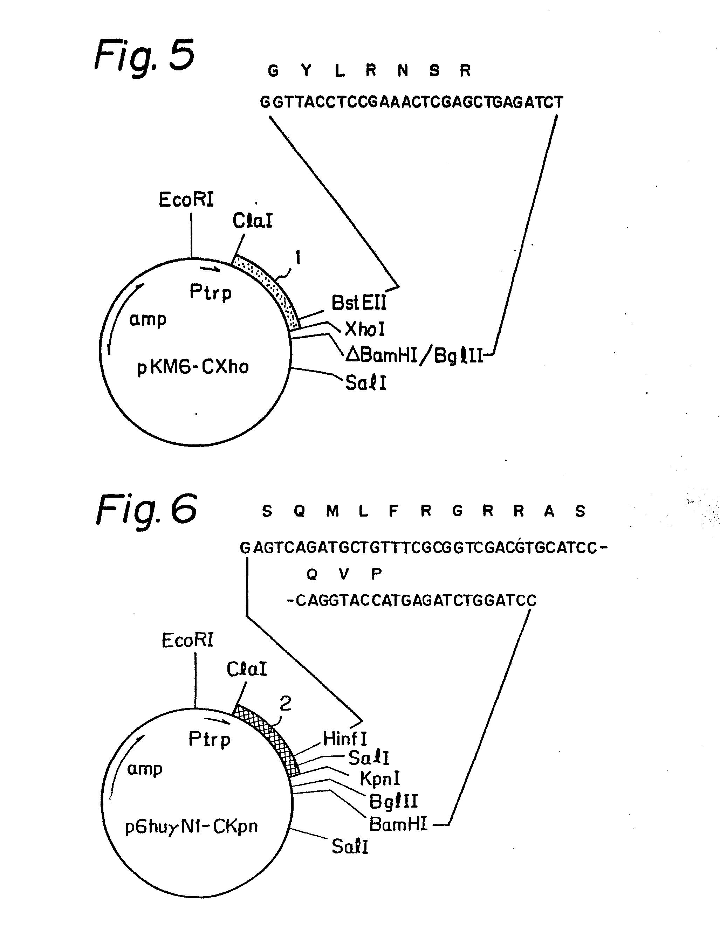

(3) Construction of plasmid pKM6-Cxho The structure of the plasmid pKM6-Cxho is shown in Fig. 5. The plasmid pKM6 constructed as described in paragraph (1) was digested with BstEII and BamHI, and an adapter oligonucleotide having the following sequence:

```
GTTACCTCCGAAACTCGAGCTGA
        GAGGCTTTGAGCTCGACTCTAG
```

was linked to the cleaved plasmid to construct the plasmid pKM6-cxho. The adapter was prepared according to the same procedure as described in paragraph (2). When the plasmid pKM6-Cxho is digested with XhoI and the protruding nucleotides are eliminated, a codon AAC coding for aspartic acid present at the C-terminal of human interferon-β is exposed.

(4) Construction of plasmid p6huγN1-CKpn

The structure of the plasmid p6huγN1-CKpn is shown in Fig. 6. The plasmid p6huγ-N1 constructed as described in paragraph (2) was digested with ClaI and BamHI, and after agarose gel electrophoresis, a DNA fragment of about 4200 bp and a DNA fragment of about 1050 bp were isolated. The DNA fragment of about 1050 bp was further digested with HinfI, and after agarose gel electrophoresis a DNA fragment of about 400 bp was isolated. The ClaI-BamHI fragment of about 4200 bp prepared as described above, the ClaI-HinfI fragment of about 400 bp, and an adapter of the following sequence:

```
(5')-AGTCAGATGCTGTTTCGCGGTCGACGTGCATCCCAGGTACCATGAGATCTG
         GTCTACGACAAAGCGCCAGCTGCACGTAGGGTCCATGGTACTCTAGACCTAG-(5')
```

were mixed and ligated, and the ligation mixture was used to transform E. coli MC1061. The above-mentioned adapter was prepared from six oligonucleotides according

to the same procedure as described in paragraph (2). The transformants selected for ampicillin resistance were subjected to colony hybridization using an oligonucleotide probe 5'-GATCCAGATCTCATG-3' to obtain four positive clones from 118 clones. The positive clones contained plasmid p6huγ-CKpn. When the thus-prepared plasmid p6huγ-CKpn is digested with KpnI, and protruding nucleotides are eliminated, a codon CAG coding for glutamine present at the C-terminal of human interferon-γ is exposed.

(5) Construction of plasmid p6huγNlΔBS-NHin

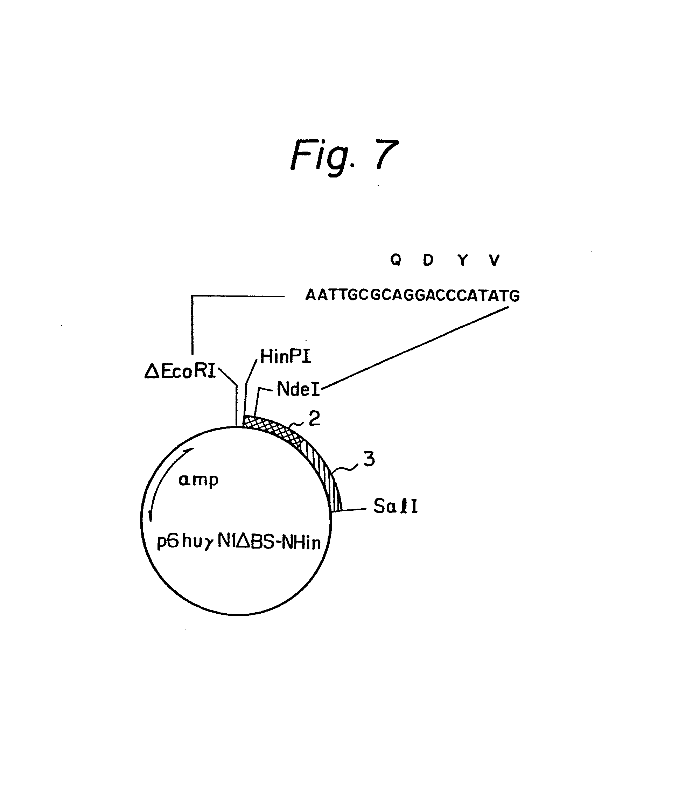

The structure of plasmid p6huγNlΔBS-NHin is shown in Fig. 7. The plasmid p6huγ-Nl constructed as described in paragraph (2) was digested with BstEII, the resulting cohesive ends were changed to blunt ends using a DNA polymerase I Klenow fragment, and the resulting DNA fragment was linked to a SalI linker. After digestion with SalI, the DNA fragment was self-circularized using the T4 DNA ligase to obtain p6huγNl-ΔBS. The plasmid p6huγNl-ΔBS was digested with NdeI and SalI, and after agarose gel electrophoresis, a DNA fragment of about 3700 bp was isolated.

On the other hand, the plasmid pKM6 constructed as described in paragraph (2) was digested with EcoRI and SalI, and after agarose gel electrophoresis, a DNA fragment of about 3700 bp was isolated.

Two DNA fragments prepared as described above were linked with an adapter oligonucleotide of the following sequence:

AATTGCGCAGGACCCA

CGCGTCCTGGGTAT

to obtain plasmid p6huγNlΔBS-NHin. When the plasmid p6huγNlΔBS-NHin is digested with HinPI, and protruding nucleotides are eliminated, a codon CAG coding for glutamine present at the N-terminal of human interferon-γ is exposed.

Example 1. Construction of plasmid ptrp6huIFN-γβ

<u>for expression of interferon-γ·β</u>
<u>conjugate</u>

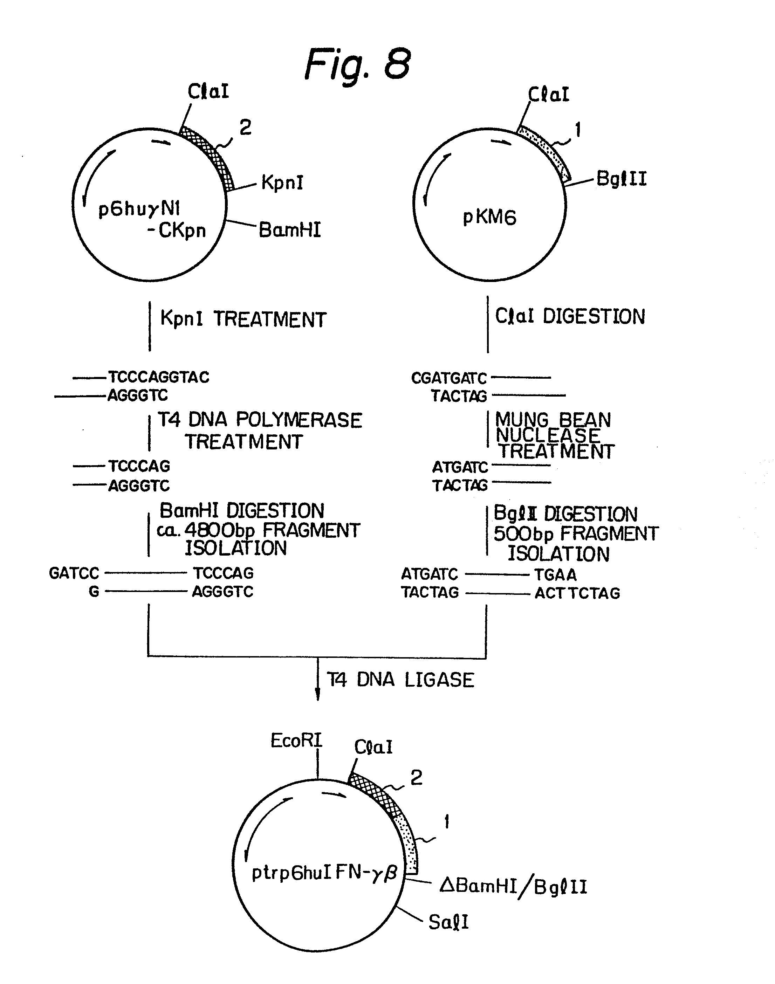

A process for the construction of a plasmid
ptrp6huIFN-γβ is shown in Fig. 8, wherein 30 µg of
plasmid pKM6 was digested with ClaI, and the digestion
product was treated with 15 units of mung bean nuclease
to change cohesive ends to blunt ends. The DNA fragment
thus-obtained was further digested with BglII, and after
agarose gel electrophoresis, a DNA fragment of about
500 bp was isolated. On the other hand, plasmid
p6huγN1-CKpn was digested with KpnI, the cohesive ends
were changed to blunt ends using T4 DNA polymerase, and
after digestion with BamHI followed by agarose gel
electrophoresis, a DNA fragment of about 4800 bp was
obtained. The DNA fragments thus-prepared were mixed
and ligated using T4 DNA ligase, and the reaction
mixture was used to transform <u>E. coli</u> HB101 (Boyer et
al., J. Mol. Biol., <u>41</u>, 459-472, 1969). The resulting
ampicillin resistant transformants were subjected to
colony hybridization using as a probe a 32P-labeled DNA
fragment prepared by nick-translating a ClaI-BglII
fragment of plasmid pKM6, to obtain 6 positive clones
among 56 clones. Plasmid DNA was extracted from each of
these clones, and a restriction enzyme cleavage map was
made. As a result, it was confirmed that plasmids from
all positive clones had a structure as shown in Fig. 8.
Moreover, plasmid DNA derived from a representative
clone γβ6 was digested with SalI, and resulting DNA
fragments were inserted to M13 phage to determine a
nucleotide sequence of the plasmid DNA. As a result, in
this plasmid, an IFN-γ structural gene was linked with
an IFN-β structural gene in frame. This desired plasmid
was designated as plasmid ptrp6huIFN-γβ, and a trans-
formant <u>E. coli</u> was designated as <u>E. coli</u> HB101
(ptrp6huIFN-γβ).

<u>Example 2.</u>  <u>Construction of plasmid ptrp6huIFN-βα</u>
<u>for expression of interferon-β·α</u>

<u>conjugate</u>

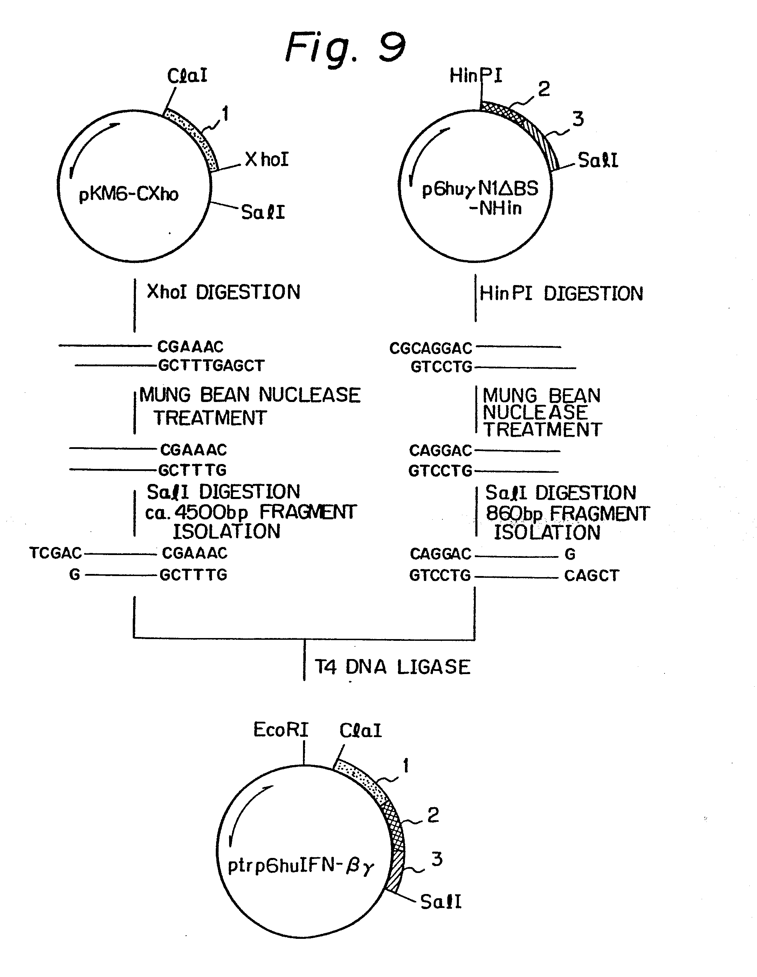

A process for the construction of a plasmid
ptrp6huIFN-βγ is shown in Fig. 9, wherein 20 µg of
plasmid pKM6-Cxho was digested with Xho I, and the
digestion product was treated with 15 units of mung bean
nuclease at 37°C for 15 minutes to make blunt ends, and
then digested with SalI.  The reaction mixture was
subjected to agarose gel electrophoresis to isolate a
DNA fragment of about 4500 bp.  On the other hand, 30 µg
of p6huγN1ΔBS-NHin was digested with HinPI, the digestion
product was treated with 30 units of mung bean nuclease
at 37°C for 15 minutes, and digested with SalI, and
after agarose gel electrophoresis a DNA fragment of
about 860 bp was isolated.

The DNA fragments thus-prepared were mixed and
ligated using the T4 DNA ligase, and the reaction
mixture was used to transform <u>E. coli</u> HB101.  Among the
resulting ampicillin resistant transformants, 50 clones
were subjected to colony hybridization using a
oligonucleotide probe 5'-AGTCAGATGCTGTTTC-3', which is
the same oligonucleotide used for the construction of
plasmid p6huγN1-CKpn, to obtain 28 positive clones.
A plasmid DNA was isolated from a representative
clone βγ31, and a restriction enzyme cleavage map was
made.  As a result, a map as shown in Fig. 9 was
obtained.  Moreover, a BstEII-SalI fragment from the
plasmid isolated from the clone βγ31 was cloned in phage
M13 to determine the nucleotide sequence.  As a result,
in the plasmid, an IFN-β structural gene was linked with
an IFN-γ structural gene in frame.  This plasmid was
designated as plasmid ptrp6huIFN-βγ, and <u>E. coli</u>
transformed with the plasmid was designated as <u>E. coli</u>
HB101 (ptrp6huIFN-βγ).

<u>Example 3.  Construction of plasmid ptrphuIFN-γcβ
for expression of interferon-γcβ
conjugate</u>

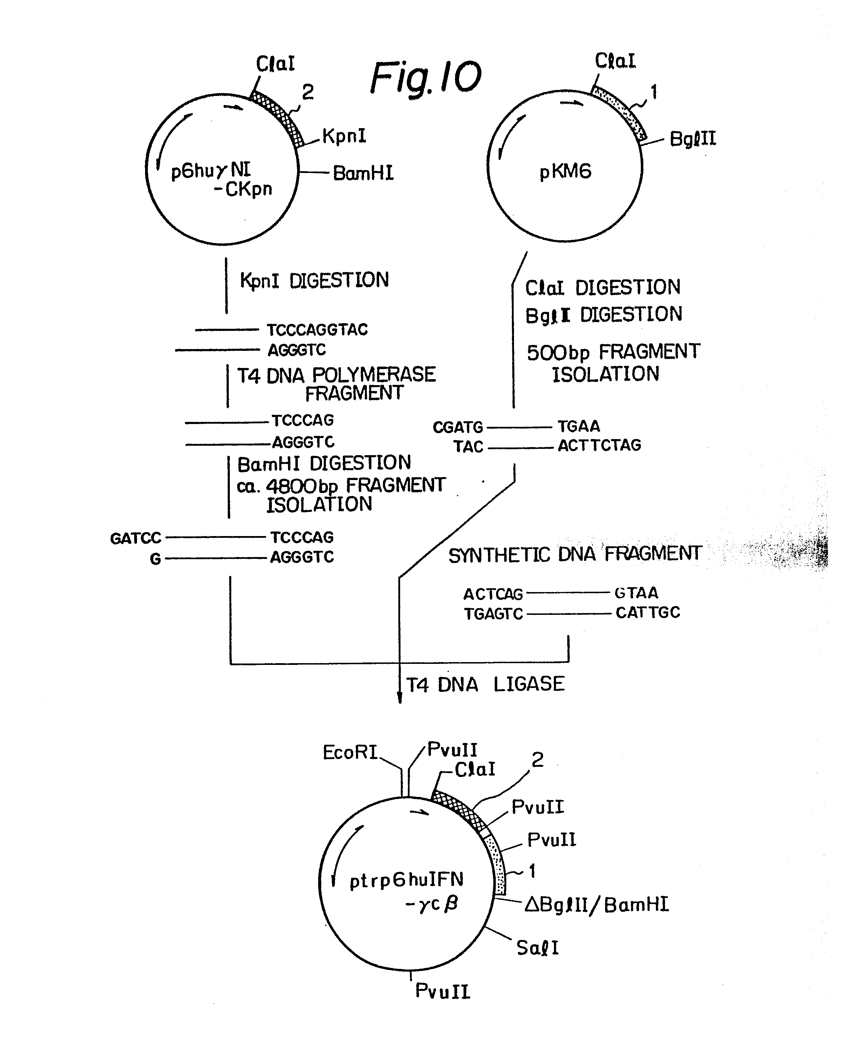

A process for the construction of a ptrphuIFN-γcβ is shown in Fig. 10. A plasmid pKM6 was digested with ClaI and then with BglII, and after agarose gel electrophoresis a DNA fragment of about 500 bp was isolated. On the other hand, a DNA fragment coding for a spacer peptide was prepared from four synthetic oligonucleotides according to the same procedure as described in paragraph (2) of the Reference Example. 10 pmoles of this DNA fragment was mixed with the ClaI-BglII fragment of pKM6 isolated as described above and the DNA fragment of about 4800 bp derived from plasmid p6huγN1-CKpn as described in Example 1, these DNA fragments were ligated using the T4 DNA ligase, and the reaction mixture was used to transform E. coli HB101. 82 clones of the resulting ampicillin resistant transformants were subjected to colony hybridization using the probe described in Example 1 to obtain three positive clones. Plasmid DNAs were isolated from the three clones, and restriction enzyme cleavage maps were made. As a result, only one clone contained a plasmid having a desired structure. This plasmid was designated as plasmid ptrp6huIFN-γcβ, and E. coli transformed with this plasmid was designated as E. coli HB101 (ptrp6huIFN-γcβ).

Example 4. Culturing and production of interferon conjugate

Transformants obtained in Examples 1 to 3 were inoculated to LB medium (1.0% bactotrypton, 0.5% yeast extract, 0.5% sodium chloride, 0.1% glucose, pH 7.2 with sodium hydroxide) supplemented with 100 μg/ml tryptophan, and 100 μg/ml ampicillin, and cultivation was carried out at 30°C for 8 hours. This culture was then inoculated to M9 medium (0.3% mono potassium phosphate, 0.6% disodium phosphate, 0.1% ammonium chloride, and 0.5% sodium chloride, and further, separately sterilized 1 μg/ml Vitamine $B_1$ and 0.1 mM magnesium sulfate) supplemented with 1.0% glucose and 1.0% casamino acids, in an amount of 10% by volume relating to a volume of the M9 medium, and cultivation was continued. After about 10 hours, indoleacrylic acid was added to the final concentration of 10 µg/ml, and cultivation was carried out for an additional 8 hours. During the cultivation, if necessary, 40% glucose aqueous solution was added to the culture medium to prevent depletion of the glucose. Moreover, during the cultivation, a 14% aqueous ammonia solution was added to the culture medium to maintain the pH value at 6.0 to 7.0.

After the cultivation, 2 ml of the cultured broth was centrifuged at 10,000X g for 4 minutes to collect cells. The cells were washed with physiological saline, and the washed cells were suspended in 1 ml of 50 mM Tris-HCl buffer (pH 7.5) containing 3 mg of lysozyme, 2 mM EDTA, 30 mM sodium chloride, and 20% glycerol, and the suspension was allowed to stand in ice for 60 minutes. The freezing and thawing of the suspension were repeated three times to disrupt the cells, and the disrupted mixture was centrifuged at 30,000X g for 20 minutes to eliminate cell debris. A supernatant thus obtained was used as a test sample for measuring.

The antiviral activity of interferon was measured according to "Interferon no Kagaku" (Science of Interferon) edited by Shigeyasu Kobayashi, p13-20, Kodan Sha, 1985, by the $CPE_{50}$ inhibition method using the FL cells-sindbis virus system. As a standard for measurement of the activity, an IFN-γ preparation was used which was produced by recombinant cells and calibrated using NIH natural IFN-γ Gg23-901-530. For reference, interferon crude extracts prepared according to the above-mentioned procedure from E. coli HB101 containing plasmid pKM6, which expresses human interferon-β, and from E. coli HB101 containing plasmid p6hur-N1, which express human interferon-γ, were tested for their antiviral activity. The results are shown in Table 1.

Table 1

| Strain | Antiviral Activity per Extract (U/ml) |
|--------|---------------------------------------|
| E. coli HB101 (ptrp6huIFN-γβ) | $3.9 \times 10^4$ |
| E. coli HB101 (ptrp6huIFN-βγ) | $1.6 \times 10^4$ |
| E. coli HB101 (ptrp6huIFN-γcβ) | $7.7 \times 10^4$ |
| E. coli HB101 (pKM6) | $3.1 \times 10^5$ |
| E. coli HB101 (p6huγ-N1) | $4.1 \times 10^4$ |

As seen from Table 1, each preparation exhibited an antiviral activity characteristic for the expected interferon.

Example 5.  Measurement of Molecular Weight

1 ml of cultured broth prepared according to the same procedure as described in Example 4 was centrifuged at 10,000X g for 4 minutes to collect cells.  The cells were suspended in 500 μl of 62.5 mM Tris-HCl buffer (pH 6.8) containing 5% 2-mercaptoethanol and 2% sodium dodecylsulfate, and the suspension was heated in a boiling water bath for 5 minutes and allowed to cool. Then, to the heat-treated suspension was added 50 μl of 62.5 mM Tris-HCl buffer (pH 6.8) containing 0.05% bromo phenol·blue and 70% glycerol, to prepare a sample for electrophoresis.  SDS-polyacrylamide gel electrophoresis was carried out according to Laemmli's method (Nature, 227, 680, 1970).  The gel concentration was 15%.  As maker proteins, lysozyme having a molecular weight of 14,400, trypsin inhibitor having a molecular weight of 21,500, carbonic anhydrase having a molecular weight of 31,000, ovalbumin having a molecular weight of 45,000, bovine serum albumin having a molecular weight of 66,200, and phosphorylase B having a molecular weight of 92,500 were used. After electrophoresis, the gel was stained with Coomasie Brilliant Blue R250 to detect proteins. Another gel obtained by simultaneous electrophoresis was used to transport proteins on the gel to a nitrocellulose sheet. The nitrocellulose sheet was reacted with commercially available anti-human interferon-β immunoglobulin or anti-human interferon-γ immunoglobulin, and then reacted with peroxidase-labeled protein A to determine the position of the interferon conjugate. By comparing a result of the above-mentioned Westean blotting with the positions of electrophoretically migrated maker proteins, it was found that IFN-γβ and IFN-βγ have a molecular weight of about 37,000, and IFN-γcβ has a molecular weight of about 38,000. That is, it was confirmed that interferon conjugates of the present invention substantially consist of human interferon-β having a molecular weight of about 20,000 and human interferon-γ having a molecular weight of about 17,000. Note, about 1000 of a molecular weight in IFN-γcβ is assigned to a spacer peptide contained in the IFN-γcβ.

Example 6. Neutralization of product with antibody

A crude interferon extract from the E. coli HB101 (ptrp6huIFN-γβ) prepared according to the procedure described in Example 4 was diluted five-fold with an Eagle's minimum essential medium containing 5% calf serum and 10 mM Hepes (pH 7.3). To 1 ml of the interferon solution thus-prepared, 1 ml of anti-IFN-β rabbit antiserum diluted 50-fold with the same medium (neutralization value 2700 U/ml) or 1 ml of anti-IFN-γ rabbit antiserum diluted 50-fold with the same medium (neutralization value 2000 U/ml) was added, and after the mixture was incubated at 37°C for 30 minutes, the

antiviral activity was measured. For control, a reaction mixture wherein the antiserum was replaced by the medium, and a reaction mixture wherein 0.5 ml of the anti-IFN-β rabbit antiserum solution and 0.5 ml of the anti-IFN-γ rabbit antiserum solution were added were prepared, and the antiviral activity was measured in same manner. The results are set forth in Table 2.

Table 2

| Antiserum | Antiviral Activity (U/ml) |
|---|---|
| Control (no antiserum) | $6.0 \times 10^3$ |
| Anti-IFN-β antiserum | $1.3 \times 10^3$ |
| Anti-IFN-γ antiserum | $1.7 \times 10^3$ |
| Anti-IFN-β antiserum + and -IFN-γ antiserum | $8.1 \times 10$ |

As seen from Table 2, each of the anti-IFN-β antiserum and the anti-IFN-γ antiserum partially neutralized antiviral activity, and a combination of anti-IFN-β antiserum and anti-IFN-γ antiserum substantially completely neutralized all antiviral activity. This means that the present interferon-γβ conjugate has both an interferon-β action and an interferon-γ action. Moreover, although $2.7 \times 10^3$ U of anti-IFN-β antiserum neutralized the IFN-β activity with $1.3 \times 10^3$ U of IFN-γ activity remaining and $2.0 \times 10^3$ U of anti-IFN-γ antiserum neutralized the IFN-γ activity with $1.7 \times 10^3$ U of IFN-β activity remaining, the not-neutralized control showed $6.0 \times 10^3$ U activity. That is, antiviral activity of the control ($6.0 \times 10^3$) is higher than total amount of remaining activities ($1.3 \times 10^3$ U + $1.7 \times 10^3$ U). This means that the present interferon-γβ conjugate exhibits synergism of IFN-β activity and IFN-γ activity.

Example 7. Construction of plasmid ptrp6huIFN-βcγ for expression of interferon βcγ

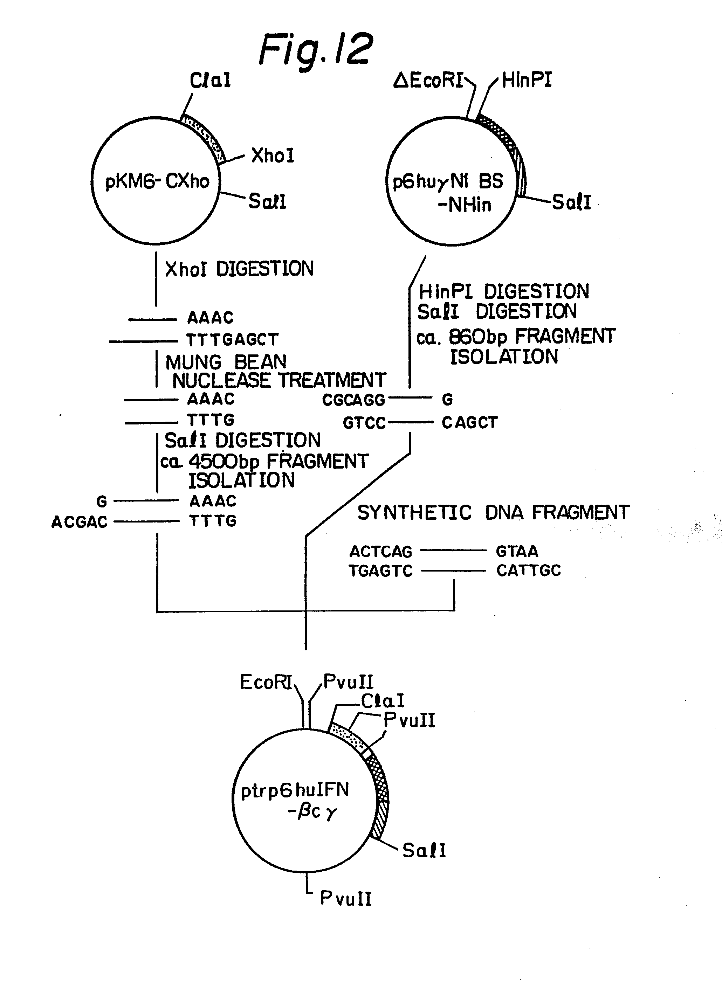

A process for the construction of a plasmid ptrp6huIFN-βcγ is shown in Fig. 12, wherein 20 µg of plasmid pKM6-Cxho was digested with XhoI, the digestion product was treated with 15 units of mung been nuclease at 37°C for 15 minutes to make blunt ends, and then digested with SalI. The reaction mixture was subjected to agarose gel electrophoresis to isolate a DNA fragment of about 4500 bp. On the other hand, 30 µg of plasmid p6huγN1ΔBS-NHin was digested with HinPI and SalI, and after agarose gel electrophoresis, a DNA fragment of about 860 bp was isolated. The above-mentioned two DNA fragments were mixed with 10 pmoles of DNA fragment coding for a spacer peptide obtained according to the procedure as described in Example 3, these three DNA fragments were ligated with T4 DNA ligase, and the ligation mixture was used to transform E. coli HB101. The 204 ampicillin resistant transformants thus obtained were subjected to colony hybridization using as a probe that shown in Example 2 or an oligonucleotide 5'-CGTTACCGACTTAGCA-3' used for preparation of the DNA fragment coding for the spacer peptide. As a result, two clones were found to be positive, and plasmids from these clones were analyzed by restriction enzymes. One clone contained a desired plasmid, and this plasmid was designated as plasmid ptrp6huIFN-βcγ, and E. coli transformed with this plasmid was designated as E. coli HB101 (ptrp6huIFN-βcγ). According to the procedures described in Example 4, the E. coli transformant was cultured and an extract prepared, and then the antiviral activity was measured. An antiviral activity of $3.9 \times 10^4$ U/ml extract was found.

Example 8. Neutralization of product with antibody

According to the same procedure as described in Example 6, a crude interferon extract from E. coli HB101

(ptrp6huIFN-γcβ) was neutralized with antisera. For comparison, an interferon mixture consisting of 8600 U/ml of IFN-β and 2400 U/ml of IFN-γ, prepared by mixing an IFN-β and an IFN-γ in approximately the same molecular numbers, were tested in the same manner. The results are shown in Table 3.

<u>Table 3</u>

| IFN | Antiserum | | Antiviral activity |
|-----|-----------|-----------|--------------------|
| | Anti-IFN-β | Anti-IFN-γ | (U/ml) |
| IFN mixture | - | - | 19000 |
| | o | - | 930 |
| | - | o | 12000 |
| | o | o | <27 |
| IFN-γcβ | - | - | 22000 |
| | o | - | 2400 |
| | - | o | 11000 |
| | o | o | 61 |

As can be seen from Table 3, in the interferon-γcβ conjugate (IFN-γcβ), each of the anti-IFN-β antiserum and anti-IFN-γ antiserum partially neutralized the antiviral activity thereof, and a combination of both antisera substantially completely neutralized all antiviral activity. This means that correctly folded IFN-β and IFN-γ molecules are liked to yield both the IFN-β activity and IFN-γ activity are exhibited by a single polypeptide.

Moreover, from the comparison of the activities resulting from neutralization with the anti-IFN-β or anti-IFN-γ with the activity resulting from neutrolization with of a combination of anti-IFN-β and anti-IFN-γ, it appears that the present IFN-γcβ conjugate also exhibits the synergism of the IFN-β activity and the IFN-γ activity.

Example 9.

A.  Construction of vector for expression of human interferon-β

pSVβ is a vector derived from the human interferon-γ expression vector pSV2IFNβ (Japanese Unexamined Patent Publication No. 61-52283) by deleting a sequence inhibiting replication of the vector in a eukaryotic cell (M. Losky et al., Nature, 293 77, 1981). The vector pSVβ was constructed as follows.

First, a PvuII site positioned in the pSV2IFNβ upstream from the SV40 early promoter was replaced by a SalI site using a SalI linker, and the modified vector was cleaved with SalI and BamHI to isolate a 1.7 Kb DNA fragment required for expression of the human interferon-β.

Next, vector pML2d (M. Lusky et al., Nature, 293, 79, 1981) derived from pBR322 by deleting a sequence inhibiting replication of the vector in the eukaryotic cell was cleaved with SalI and BamHI to isolate a long DNA fragment.

These two DNA fragments were ligated using T4 DNA ligase to obtain pSVβ.

B.  Construction of vector pMTVβ for expression of human interferon-β

The vector pSVβ constructed in Section A was cleaved with SalI, and the SalI site in the vector was replaced by a HindIII site using a HindIII linker, and the modified vector was cleaved with HindIII to isolate a 3.8 Kb DNA fragment which does not contain the SV40 early promoter.  The DNA fragment thus obtained was then treated with E. coli alkaline phosphatase to eliminate a terminal phosphate group.

Next, vector pMTVdhfr (F. Lee et al., Nature,

<u>294</u>, 228, 1982) containing MMTV promoter was cleaved with a restriction enzyme HindIII to isolate a 1.4 Kb DNA fragment containing MMTV promoter.

These two DNA fragments were ligated using T4 DNA ligase to obtain pMTVβ.

C. <u>Construction of vector pMTVγ for expression of human interferon-γ</u>

pMTVγ is a vector wherein a human interferon-γ gene is positioned under control by an MMTV promoter.

The vector pMTVβ constructed in Section B was cleaved at a HindIII site downstream from the MMTV promoter and at the BglII site downstream from the human interferon gene, and after the resulting fragments were blunt-ended using a DNA polymerase I Klenow fragment, a 3.9 Kb DNA fragment containing the MMTV promoter was isolated.

This DNA fragment was ligated with a 0.8 Kb DNA fragment containing a human interferon-γ gene, which was obtained from pSVIFNγ (Japanese Unexamined Patent Publication No. 61-52286) by cleaving with DpnI, using a T4 DNA ligase to obtain pMTVγ.

D. <u>Construction of vector pMTV(SV)γ for expression of human interferon-γ</u>

pMTV(SV)γ is a vector derived from pMTVγ by inserting an SV40 early promoter upstream of the MMTV promoter. The vector pMTV(SV)γ was constructed as follows.

pMTVγ constructed in Section C was cleaved with SalI, and after the resulting DNA fragment was blunt-ended using a DNA polymerase I Klenow fragment, a terminal phosphate group was eliminated by BAP treatment.

Next, pSV2IFNβ (Japanese Unexamined Patent Publication 61-52283) was cleaved with PvuII and HindIII to isolate a 0.3 Kb DNA fragment containing the SV40 early promoter, and the fragment was blunt-ended by treating with a DNA polymerase I Klenow fragment.

These two DNA fragments were ligated using T4 DNA ligase to obtain pMTV(SV)γ.

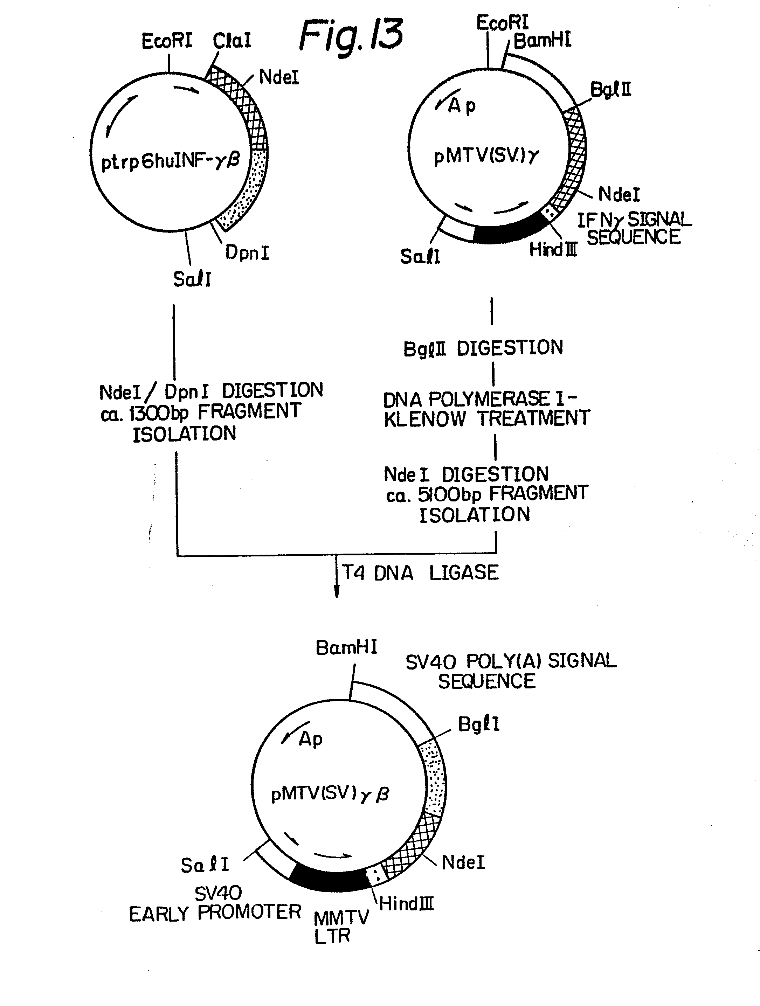

E.    Construction of plasmid pMTV(SV)γ·β for expression of human interferon-γ·β conjugate in animal cells (Fig. 13)

pMTV(SV)γ·β is a vector wherein the human interferon-γ gene in pMTV(SV)γ has been replaced by the human interferon-γ·β conjugate gene, and was constructed as follows.

10 μg of ptrp6huIFN-γβ constructed according to the procedure as described in Example 1 was digested with NdeI and DpnI, and the digestion mixture was subjected to agarose gel electrophoresis to isolate a DNA fragment of about 1300 bp.

On the other hand, pMTV(SV)γ constructed in Section D was digested with BglII, and the resulting fragment was blund-ended by treating with DNA polymerase I Klenow fragment, followed by digestion with NdeI, and the reaction mixture was subjected to agarose gel electrophoresis to isolate a DNA fragment of about 5100 bp.

These DNA fragments were mixed and ligated with T4 DNA ligase to obtain pMTV(SV)γ·β.

Example 10.    Construction of plasmid pMTV(SV)γcβ for expression of human interferon-γcβ conjugate in animal cells

Plasmid pMTV(SV)γcβ is a vector wherein a human interferon-γ gene in pMTV(SV)γ has been replaced by a human interferon-γcβ conjugate gene, and was constructed as follows.

10 μg of ptrp6huIFN-γcβ constructed according to the procedure as described in Example 3 was digested with NdeI and DpnI, and the reaction mixture was subjected to agarose gel electrophoresis to isolate a DNA fragment of about 1300 bp.  On the other hand, pMTV(SV)γ constructed in Section D was digested with BglII, and the resulting DNA fragment was blunt-ended by treating with a DNA polymerase I Klenow fragment, followed by digestion with NdeI, and the reaction mixture was subjected to agarose gel electrophoresis to isolate a DNA fragment of about 5100 bp.

These DNA fragments were mixed, and ligated using T4 DNA ligase to obtain pMTV(SV)γcβ.

Example 11.  Transformation of PC12 cell with MTV(SV)γ·β

4 µg of vector pMTV(SV)γ·β constructed in Example 9 and 0.4 µg of GC18 resistance gene expression vector pSV2neo (J. Southern et al., J. Mo. Appl. Genet., $\underline{1}$, 327, 1982) were introduced into about $10^6$ cells of PC12 cell line (M. Kinjo et al., Br. J. Cancer, $\underline{39}$, 15, 1979) derived from human lung cancer, according to the calcium phosphate method.  The cells thus-prepared were cultured in a selective medium (RPMI 1640 medium supplemented with 10% fetal calf serum and 100 µg/ml kanamycine; Nissui Seiyaku, Japan) containing 400 µg/ml of a protein synthesis inhibitor, GC18 (GIBCO) to obtain 24 transformant clones.  The antiviral activity of the cultured supernatants was tested using FL cells-sindbis virus system according to the $CPE_{50}$ inhibition method described in Example 4.  As a result, supernatants from 22 clones exhibited the antiviral activity, as shown in Table 4.

- 32 -

0237019

## Table 4

| pMTV(SV) $\gamma \cdot \beta$/PC12 | |
|---|---|
| Clone | Antiviral activity (U/ml) |
| 1 | 18500 |
| 2 | 1100 |
| 3 | 600 |
| 4 | 1500 |
| 5 | < 80 |
| 6 | 1300 |
| 7 | 200 |
| 8 | 2300 |
| 9 | 200 |
| 10 | < 80 |
| 11 | 1000 |
| 12 | 2500 |
| 13 | 900 |
| 14 | 1400 |
| 15 | 500 |
| 16 | 400 |
| 17 | < 80 |
| 18 | < 80 |
| 19 | 300 |
| 20 | 800 |
| 21 | 200 |
| 22 | 300 |
| 23 | 200 |
| 24 | 600 |

## Example 12. Transformation of PC12 cells with pMTV(SV)γcβ

4 μg of pMTV(SV)γcβ constructed in Example 10 and 0.4 μg of pSV2neo (see Example 11) were introduced into about $10^6$ cells of the PC12 cell line by a calcium phosphate method according to the same procedure as described in Example 11. The cells thus-prepared were cultured in a selective medium (RPMI 1640 medium supplemented with 10% fetal calf serum and 100 μg/ml kanamycine; Nissui Seiyaku, Japan) to obtain 26 transformed clones. The antiviral activity of the cultured supernatants was tested by the $CPE_{50}$ inhibition method using the FL cells-sindbis virus system according to the same procedure as described in Example 11. As a result, supernatants from 20 clones exhibited the antiviral activity as shown in Table 5.

Table 5

| pMTV(SV)γcβ/PC12 | |
| --- | --- |
| Clone | Antiviral activity (U/ml) |
| 1 | 400 |
| 2 | < 60 |
| 3 | < 60 |
| 4 | 3800 |
| 5 | 1200 |
| 6 | < 60 |
| 7 | 6400 |
| 8 | 200 |
| 9 | < 80 |
| 10 | 400 |
| 11 | 200 |
| 12 | 900 |
| 13 | 500 |
| 14 | 700 |
| 15 | < 80 |
| 16 | 1300 |
| 17 | 21450 |
| 18 | 130 |
| 19 | 5300 |
| 20 | 1600 |
| 21 | 200 |
| 22 | < 80 |
| 23 | 1200 |
| 24 | 8600 |
| 25 | 300 |
| 26 | 500 |

## CLAIMS

1.    An interferon conjugate comprising in a single molecule a region exhibiting biological activities of interferon-β and a region exhibiting biological activities of interferon-γ.

2.    An interferon conjugate according to claim 1, which is a biologically active polypeptide having no glycoside chain.

3.    An interferon conjugate according to claim 1, which is a biologically active polypeptide having glycoside chains.

4.    An interferon conjugate according to any one of claims 1 to 3, wherein the region exhibiting biological activities of interferon-β and the region exhibiting biological activities of interferon-γ have been linked via a spacer.

5.    An interferon conjugate according to claim 4, wherein the spacer is a polypeptide.

6.    An interferon conjugate according to any one of claims 1 to 3, wherein a C-terminal of the region exhibiting biological activities of interferon-β has been linked to an N-terminal of the region exhibiting biological activities of interferon-γ, directly or via a spacer.

7.    An interferon conjugate according to claim 6, wherein the interferon-β consists of the following amino acid sequence:

                MET SER TYR ASN LEU LEU GLY PHE LEU GLN ARG SER SER
ASN PHE GLN CYS GLN LYS LEU LEU TRP GLN LEU ASN GLY ARG LEU GLU TYR
CYS LEU LYS ASP ARG MET ASN PHE ASP ILE PRO GLU GLU ILE LYS GLN LEU
GLN GLN PHE GLN LYS GLU ASP ALA ALA LEU THR ILE TYR GLU MET LEU GLN
ASN ILE PHE ALA ILE PHE ARG GLN ASP SER SER SER THR GLY TRP ASN GLU
THR ILE VAL GLU ASN LEU LEU ALA ASN VAL TYR HIS GLN ILE ASN HIS LEU
LYS THR VAL LEU GLU GLU LYS LEU GLU LYS GLU ASP PHE THR ARG GLY LYS
LEU MET SER SER LEU HIS LEU LYS ARG TYR TYR GLY ARG ILE LEU HIS TYR
LEU LYS ALA LYS GLU TYR SER HIS CYS ALA TRP THR ILE VAL ARG VAL GLU
ILE LEU ARG ASN PHE TYR PHE ILE ASN ARG LEU THR GLY TYR LEU ARG ASN,

and the interferon-γ consists of the following amino
acid sequence:

GLN ASP PRO TYR VAL LYS GLU ALA GLU ASN LEU LYS LYS
TYR PHE ASN ALA GLY HIS SER ASP VAL ALA ASP ASN GLY THR LEU PHE LEU
GLY ILE LEU LYS ASN TRP LYS GLU GLU SER ASP ARG LYS ILE MET GLN SER
GLN ILE VAL SER PHE TYR PHE LYS LEU PHE LYS ASN PHE LYS ASP ASP GLN
SER ILE GLN LYS SER VAL GLU THR ILE LYS GLU ASP MET ASN VAL LYS PHE
PHE ASN SER ASN LYS LYS LYS ARG ASP ASP PHE GLU LYS LEU THR ASN TYR
SER VAL THR ASP LEU ASN VAL GLN ARG LYS ALA ILE HIS GLU LEU ILE GLN
VAL MET ALA GLU LEU SER PRO ALA ALA LYS THR GLY LYS ARG LYS ARG SER
GLN MET LEU PHE ARG GLY ARG ARG ALA SER GLN.

8.     An interferon conjugate according to claim 7,
wherein a C-terminal of a polypeptide consisting of the
following amino acid sequence:

MET SER TYR ASN LEU LEU GLY PHE LEU GLN ARG SER SER
ASN PHE GLN CYS GLN LYS LEU LEU TRP GLN LEU ASN GLY ARG LEU GLU TYR
CYS LEU LYS ASP ARG MET ASN PHE ASP ILE PRO GLU GLU ILE LYS GLN LEU
GLN GLN PHE GLN LYS GLU ASP ALA ALA LEU THR ILE TYR GLU MET LEU GLN
ASN ILE PHE ALA ILE PHE ARG GLN ASP SER SER SER THR GLY TRP ASN GLU
THR ILE VAL GLU ASN LEU LEU ALA ASN VAL TYR HIS GLN ILE ASN HIS LEU
LYS THR VAL LEU GLU GLU LYS LEU GLU LYS GLU ASP PHE THR ARG GLY LYS
LEU MET SER SER LEU HIS LEU LYS ARG TYR TYR GLY ARG ILE LEU HIS TYR
LEU LYS ALA LYS GLU TYR SER HIS CYS ALA TRP THR ILE VAL ARG VAL GLU
ILE LEU ARG ASN PHE TYR PHE ILE ASN ARG LEU THR GLY TYR LEU ARG ASN
has been linked to an N-terminal of a polypeptide
consisting of the following amino acid sequence:

GLN ASP PRO TYR VAL LYS GLU ALA GLU ASN LEU LYS LYS
TYR PHE ASN ALA GLY HIS SER ASP VAL ALA ASP ASN GLY THR LEU PHE LEU
GLY ILE LEU LYS ASN TRP LYS GLU GLU SER ASP ARG LYS ILE MET GLN SER
GLN ILE VAL SER PHE TYR PHE LYS LEU PHE LYS ASN PHE LYS ASP ASP GLN
SER ILE GLN LYS SER VAL GLU THR ILE LYS GLU ASP MET ASN VAL LYS PHE
PHE ASN SER ASN LYS LYS LYS ARG ASP ASP PHE GLU LYS LEU THR ASN TYR
SER VAL THR ASP LEU ASN VAL GLN ARG LYS ALA ILE HIS GLU LEU ILE GLN
VAL MET ALA GLU LEU SER PRO ALA ALA LYS THR GLY LYS ARG LYS ARG SER
GLN MET LEU PHE ARG GLY ARG ARG ALA SER GLN,
via a polypeptide as a spacer.

9.     An interferon conjugate according to any one of claims 1 to 3, wherein a C-terminal of the region exhibiting biological activities of interferon-γ has been linked to an N-terminal of the region exhibiting biological activities of interferon-β, directly or via a spacer.

10. An interferon conjugate according to claim 9, wherein the interferon-γ consists of the following amino acid sequence:

GLN ASP PRO TYR VAL LYS GLU ALA GLU ASN LEU LYS LYS TYR PHE ASN ALA GLY HIS SER ASP VAL ALA ASP ASN GLY THR LEU PHE LEU GLY ILE LEU LYS ASN TRP LYS GLU GLU SER ASP ARG LYS ILE MET GLN SER GLN ILE VAL SER PHE TYR PHE LYS LEU PHE LYS ASN PHE LYS ASP ASP GLN SER ILE GLN LYS SER VAL GLU THR ILE LYS GLU ASP MET ASN VAL LYS PHE PHE ASN SER ASN LYS LYS LYS ARG ASP ASP PHE GLU LYS LEU THR ASN TYR SER VAL THR ASP LEU ASN VAL GLN ARG LYS ALA ILE HIS GLU LEU ILE GLN VAL MET ALA GLU LEU SER PRO ALA ALA LYS THR GLY LYS ARG LYS ARG SER GLN MET LEU PHE ARG GLY ARG ARG ALA SER GLN,
and the interferon-β consists of the following amino acid sequence:

MET SER TYR ASN LEU LEU GLY PHE LEU GLN ARG SER SER ASN PHE GLN CYS GLN LYS LEU LEU TRP GLN LEU ASN GLY ARG LEU GLU TYR CYS LEU LYS ASP ARG MET ASN PHE ASP ILE PRO GLU GLU ILE LYS GLN LEU GLN GLN PHE GLN LYS GLU ASP ALA ALA LEU THR ILE TYR GLU MET LEU GLN ASN ILE PHE ALA ILE PHE ARG GLN ASP SER SER SER THR GLY TRP ASN GLU THR ILE VAL GLU ASN LEU LEU ALA ASN VAL TYR HIS GLN ILE ASN HIS LEU LYS THR VAL LEU GLU GLU LYS LEU GLU LYS GLU ASP PHE THR ARG GLY LYS LEU MET SER SER LEU HIS LEU LYS ARG TYR TYR GLY ARG ILE LEU HIS TYR LEU LYS ALA LYS GLU TYR SER HIS CYS ALA TRP THR ILE VAL ARG VAL GLU ILE LEU ARG ASN PHE TYR PHE ILE ASN ARG LEU THR GLY TYR LEU ARG ASN.

11. An interferon conjugate according to claim 10, wherein a C-terminal of a polypeptide consisting of the following amino acid sequence:

GLN ASP PRO TYR VAL LYS GLU ALA GLU ASN LEU LYS LYS TYR PHE ASN ALA GLY HIS SER ASP VAL ALA ASP ASN GLY THR LEU PHE LEU GLY ILE LEU LYS ASN TRP LYS GLU GLU SER ASP ARG LYS ILE MET GLN SER GLN ILE VAL SER PHE TYR PHE LYS LEU PHE LYS ASN PHE LYS ASP ASP GLN SER ILE GLN LYS SER VAL GLU THR ILE LYS GLU ASP MET ASN VAL LYS PHE

PHE ASN SER ASN LYS LYS LYS ARG ASP ASP PHE GLU LYS LEU THR ASN TYR
SER VAL THR ASP LEU ASN VAL GLN ARG LYS ALA ILE HIS GLU LEU ILE GLN
VAL MET ALA GLU LEU SER PRO ALA ALA LYS THR GLY LYS ARG LYS ARG SER
GLN MET LEU PHE ARG GLY ARG ARG ALA SER GLN

has been linked to an N-terminal of a polypeptide
consisting of the following amino acid sequence:

MET SER TYR ASN LEU LEU GLY PHE LEU GLN ARG SER SER
ASN PHE GLN CYS GLN LYS LEU LEU TRP GLN LEU ASN GLY ARG LEU GLU TYR
CYS LEU LYS ASP ARG MET ASN PHE ASP ILE PRO GLU GLU ILE LYS GLN LEU
GLN GLN PHE GLN LYS GLU ASP ALA ALA LEU THR ILE TYR GLU MET LEU GLN
ASN ILE PHE ALA ILE PHE ARG GLN ASP SER SER SER THR GLY TRP ASN GLU
THR ILE VAL GLU ASN LEU LEU ALA ASN VAL TYR HIS GLN ILE ASN HIS LEU
LYS THR VAL LEU GLU GLU LYS LEU GLU LYS GLU ASP PHE THR ARG GLY LYS
LEU MET SER SER LEU HIS LEU LYS ARG TYR TYR GLY ARG ILE LEU HIS TYR
LEU LYS ALA LYS GLU TYR SER HIS CYS ALA TRP THR ILE VAL ARG VAL GLU
ILE LEU ARG ASN PHE TYR PHE ILE ASN ARG LEU THR GLY TYR LEU ARG ASN,

via a polypeptide as a spacer

12. A deoxyribonucleotide sequence coding for an
interferon conjugate comprising in a single molecule a
region exhibiting biological activities of interferon-β
and a region exhibiting biological activities of
interferon-γ.

13. A recombinant DNA comprising a deoxyribonucleotide sequence coding for an interferon conjugate
comprising in a single molecule a region exhibiting
biological activities of interferon-β and a region
exhibiting biological activities of interferon-γ, and a
deoxyribonucleotide sequence coding for a control region
for expression of said interferon conjugate, wherein the
latter sequence is upstream from the former sequence.

14. A transformant organism transformed with a
recombinant DNA comprising a deoxyribonucleotide sequence
coding for an interferon conjugate comprising in a
single molecule a region exhibiting biological activities
of interferon-β and a region exhibiting biological
activities of interferon-γ, and a deoxyribonucleotide
sequence coding for a control region for expression of said interferon conjugate, wherein the latter sequence is upstream from the former sequence.

15. A process for production of an interferon conjugate comprising in a single molecule a region exhibiting biological activities of interferon-β and a region exhibiting biological activities of interferon-β, comprising the step:

        culturing a transformant organism transformed with a recombinant DNA comprising a deoxyribonucleotide sequence coding for said interferon conjugate and a deoxyribonucleotide sequence coding for a control region for expression of the interferon conjugate wherein the latter sequence is upstream from the former sequence, to produce the interferon conjugate;

        and recovering said interferon conjugate.

16. A process according to claim 15, wherein E. coli is used as a host.

17. A process according to claim 11, wherein human cells are used as a host.

18. A process according to claim 17, wherein the human cells are cells derived from human lung cancer.

19. A process according to claim 18, wherein the cells derived from human lung cancer are selected from the group consisting of PC8 and PC12 cell lines.

# Fig. 1

MET SER TYR ASN

LEU LEU GLY PHE LEU GLN ARG SER SER ASN PHE GLN CYS GLN LYS LEU LEU TRP GLN LEU ASN GLY ARG LEU GLU

TYR CYS LEU LYS ASP ARG MET ASN PHE ASP ILE PRO GLU GLU ILE LYS GLN LEU GLN GLN PHE GLN LYS GLU ASP

ALA ALA LEU THR ILE TYR GLU MET LEU GLN ASN ILE PHE ALA ILE PHE ARG GLN ASP SER SER SER THR GLY TRP

ASN GLU THR ILE VAL GLU ASN LEU LEU ALA ASN VAL TYR HIS GLN ILE ASN HIS LEU LYS THR VAL LEU GLU GLU

LYS LEU GLU LYS GLU ASP PHE THR ARG GLY LYS LEU MET SER SER LEU HIS LEU LYS ARG TYR TYR GLY ARG ILE

LEU HIS TYR LEU LYS ALA LYS GLU TYR SER HIS CYS ALA TRP THR ILE VAL ARG VAL GLU ILE LEU ARG ASN PHE

TYR PHE ILE ASN ARG LEU THR GLY TYR LEU ARG ASN

1/12

## Fig. 2

GLY ASP PRO TYR VAL LYS GLU ALA GLU ASN LEU LYS LYS TYR PHE ASN ALA GLY HIS SER ASP VAL

ALA ASP ASN GLY THR LEU PHE LEU GLY ILE LEU LYS ASN TRP LYS GLU GLU SER ASP ARG LYS ILE MET GLN SER

GLN ILE VAL SER PHE TYR PHE LYS LEU PHE LYS ASN PHE LYS ASP ASP GLN SER ILE GLN LYS SER VAL GLU THR

ILE LYS GLU ASP MET ASN VAL LYS PHE PHE ASN SER ASN LYS LYS LYS ARG ASP ASP PHE GLU LYS LEU THR ASN

TYR SER VAL THR ASP LEU ASN VAL GLN ARG LYS ALA ILE HIS GLU LEU ILE GLN VAL MET ALA GLU LEU SER PRO

ALA ALA LYS THR GLY LYS ARG LYS ARG SER GLN MET LEU PHE ARG GLY ARG ARG ALA SER GLN

# Fig. 3

M
AACTAGTACGCAAGTTCACGTAAAAAGGGTTTGAAATCGATG

HpaI

EcoRI

CIaI

1

Ptrp

BstEII
BgIII

amp

BamHI

pKM6   tet

SaII

# Fig. 4

CIaI

EcoRI

NdeI

2

Ptrp

HinfI

HinfI

3

amp

BstEII

p6huγ-N1

HinPI

BamHI

SaII

## Fig. 5

G  Y  L  R  N  S  R

GGTTACCTCCGAAACTCGAGCTGAGATCT

EcoRI
ClaI
Ptrp
amp
pKM6-CXho

BstEII
XhoI
ΔBamHI/BgⅡ
SalI

## Fig. 6

S  Q  M  L  F  R  G  R  R  A  S

GAGTCAGATGCTGTTTCGCGGTCGACGTGCATCC-

Q   V   P

-CAGGTACCATGAGATCTGGATCC

EcoRI
ClaI
Ptrp
amp
p6huγN1-CKpn

HinfI
SalI
KpnI
BgⅡ
BamHI
SalI

*Fig. 7*

# Fig. 8

# Fig. 9

# Fig.10

# Fig. 11

```
T  Q  L  G  Q  P  K  A  A  K  S  V  T

ACTCAGCTGGGCCAGCCGAAAGCTGCTAAGTCGGTAA

TGAGTCGACCCGGTCGGCTTTCGAGCATTCAGCCATTGC
   PvuII
```

## Fig.12

Fig. 13

## Fig. 14